# EUROPEAN PATENT SPECIFICATION

(11) **EP 1 578 953 B1**
(45) Date of publication and mention of the grant of the patent: **02.03.2011**
(21) Application number: 03812536.5
(22) Date of filing: 08.12.2003
(51) Int. Cl.: C12N 9/64

(54) **INHIBITORS OF ENDOOLIGOPEPTIDASE A (EOPA) ENZYME ACTIVITY AND THEIR USES**
INHIBITOREN DER ENZYMAKTIVITÄT DER ENDOOLIGOPEPTIDASE A (EOPA) UND IHRE VERWENDUNGEN
INHIBITEURS DE L'ACTIVITE ENZYMATIQUE DE L'ENDOOLIGOPEPTIDASE A (EOPA) ET LEURS UTILISATIONS

(30) Priority: 09.12.2002 BR 0205000; 05.12.2003 BR 00305688
(43) Date of publication of application: 28.09.2005
(73) Proprietor: Camargo, Antônio, 01453-010 Sao Paulo (BR)
(72) Inventor: CAMARGO, Antônio, 01453-010 Sao Paulo (BR); HAYASHI, Mirian, 01403-020 Sao Paulo (BR); PORTARO, Fernanda, 05599-140 Sao Paulo (BR); GUERREIRO, Juliano, 13421-470 Piracicaba (BR)
(74) Representative: Alves Moreira, Pedro
(86) International application number: PCT/BR2003/000189
(87) International publication number: WO 2004/053052

(56) References cited:
- US-B1- 6 406 891
- DATABASE EMBL 27 April 2001 (2001-04-27), HAYASHI M. A. ET AL.: "Homo sapiens endooligopeptidase A mRNA, complete cds." XP002436344 Database accession no. AF217798
- HAYASHI M A ET AL: "Molecular and immunochemical evidences demonstrate that endooligopeptidase A is the predominant cytosolic oligopeptidase of rabbit brain." BIOCHEMICAL AND BIOPHYSICAL RESEARCH COMMUNICATIONS, vol. 269, no. 1, 5 March 2000 (2000-03-05), pages 7-13, XP002436337 ISSN: 0006-291X
- MEDEIROS M S ET AL: "Distribution and properties of endo-oligopeptidases A and B in the human neuroendocrine system." THE JOURNAL OF ENDOCRINOLOGY, vol. 135, no. 3, December 1992 (1992-12), pages 579-588, XP009084802 ISSN: 0022-0795
- SHRIMPTON C N ET AL: "Development and characterization of novel potent and stable inhibitors of endopeptidase EC 3.4.24.15." THE BIOCHEMICAL JOURNAL, vol. 345 Pt 2, 15 January 2000 (2000-01-15), pages 351-356, XP002436339 ISSN: 0264-6021
- SWEENEY K J ET AL: "NudE-L, a novel Lis1-interacting protein, belongs to a family of vertebrate coiled-coil proteins." MECHANISMS OF DEVELOPMENT, vol. 101, no. 1-2, March 2001 (2001-03), pages 21-33, XP002436340 ISSN: 0925-4773
- CARVALHO ET AL.: 'Purification of Rabbitt Brain Endooligopeptidases and Preparation of Anti-Enzyme Antibodies' BIOCHEMISTRY vol. 20, no. 25, December 1981, pages 7082 - 7088, XP003002380
- DAVIS ET AL.: 'Section 11: Preparation and Analysis of RNA from Eukaryotic Cells. Basic Methods in Molecular Biology', 1986, ELSEVIER SCIENCE PUBLISHING CO., INC. NEW YORK, NEW YORK pages 129 - 156, XP008070863

## Description

The disclosure relates to the human recombinant endooligopeptidase A (hEOPA), to the polynucleotides coding for HEOPA, which allow the expression of the HEOPA in prokaryotes and eukaryotes, including in human beings; to the utilization of the synthetic substrates for the determination of the proteolytic activity of HEOPA, or for the evaluation of its activity as chaperone or as peptides "soluble receptor"; to the obtaining and utilization of specific antibodies and inhibitors of its oligopeptide-ligand activity that can act as agonists, competitors and antagonists, able to influence on its interaction with other proteins and on the formation of multiprotein complexes. The invention also relates to the application of this protein, in its native, recombinant, chemically or genetically modified forms, for diagnosis and/or application in congenital, infectious and degenerative pathologies of the central nervous system, and for psychiatric, cognitive, and behavioral dysfunctions. It also proposes the application of inhibitors and competitors of the interaction of ligands to the EOPA, including antibodies and their derivatives for the treatment of neurological pathologies and tissue degenerations.

More specifically, the disclosure relates to a protein expressed in the central nervous system (CNS), which shows proteolytic activity against peptides substrates, and displays a protein structure that allows its interaction with oligopeptides and/or intracellular proteins, determining important physiological functions like neuronal cell migration, neuritogenesis, interneuronal signaling (e.g., signaling for synapses formation), antigen presentation, and biotransformation and/or protection of bioactive peptides (chaperone activity), or modulating protein interactions of bioactive peptides ("soluble receptor" activity).

A specific methodology is proposed to determine the primary structure of the messenger RNA coding for this protein and, therefore, also the primary structure of the protein sequence of the human EOPA.

Likewise, the present disclosure relates to the determination of the primary structure of the EOPA gene, and also of the regulatory factors controlling its transcription and expression.

In addition, the primary specificity of the EOPA was characterized, regarding to the substrate hydrolysis (proteolytic activity) and also to the interaction with peptides in its active center (chaperone or "soluble receptor"-like activity), thereby influencing the formation of active complexes, able to act in cellular processes of the central nervous system, and generating active products. It also relates to the generation and/or application of synthetic or natural specific inhibitors, which might be used "in vitro", "ex vivo", or "in vivo", influencing EOPA's physiological or pathological role.

The generation of specific antibodies is described, which are able to both inhibit the catalytic activity of EOPA and to recognize EOPA specifically in tissues and biological fluids.

Further described is the use of either the polynucleotide encoding for the EOPA, the respective recombinant protein or the antibody generated against it for the determination of the distribution pattern of this protein in different tissues, organs and fluids of humans and animals.

Further described is the determination of the expression and secretion of this protein in tumor tissues or cells; in totipotent (stem cells) and neuronal cells, during differentiation process, formation of neurites or in the process of synaptic connection formation; and also in pathological processes, degenerative or repair thereof, especially of the nervous tissue and smooth muscle.

Cloning of the cDNA coding for the human EOPA and analysis of its deduced amino acid sequence

The following steps were undertaken to determine the primary structure of the messenger RNA coding for the mammalian EOPA and the corresponding protein sequence:
a) total RNA was isolated from several animal tissues, employing the guanidine isothiocyanide-phenol-chloroform extraction method or, alternatively, using Trizol™ (GibcoBRL);
   The cDNA coding for the human EOPA was isolated from a human brain cortex cDNA library, purchased from Stratagene (La Jolla, USA), after screening by hybridizations employing radioactive-labeled probes derived from a cDNA sequence coding for the rabbit EOPA, identified and described by the authors of the present invention (Hayashi et al. , 2000), and deposited at the GenBank databank under the Acc. No. AF015037 
(cctgacgtgtcggggaggagccgggtgccgaggtgcgcggagtggagctcagggctgc
ggagggaagcggagctgagcggctggggcggcctggccaggccagcggagctgaggcgt
cggtcagccggcggcgaacatgcgctttgacacattggaggctttcttgatcatggat
ggtgaagatataccagattttcaagtttaaaggaggaaactgcttattggaaggaact
ttccttgaagtataagcaaacgttccaggaagctcgggatgagctagttgaattccagg
aaggaagcagagaattagaagcagagttggaggcacaattagtacaggctgaacaaaga
aatagagacttgcaagctgataaccaaagactgaaatatgaagtggaggcgctaaagga
gaaactggaacatcagtacgcacagagctacaagcaggtttccgtattagaagatgatt
taagtcagactcgggccattaaggagcagttgcacaagtacgtgagggagctggagcag
gccaatgatgacctggagcgagcaaaaagggcaacaatagtttcactggaagactttga
acaaaggctaaatcaggccatagaacggaatgcattttttagaaagtgaacttgatgaaa
aggaatctttgttggtctctgtacagcggttaaaggatgaagcaagagatttgaggcaa
gaactagcagtccgagaaagacaacaggaagtgaccagaaagtcggctcctagttctcc
aactctagactgtgaaaagatggattctgctgtacaagcatcactttccttgccagcta
cacctgttggcaaagggacagaaaacagttttccttccccaaaagctataccaaatggt
tttggtaccagtccactaactccttcggccaggatatcagcgctaaacattgtgggga
tctcttacggaaagtaggggctttagaatccaaattagcagcttgcaggaattttgcaa
aggaccaagcatcacggaaatcctatatttcagggaatgttaactgtggggtgatgaac
agcaacggcacaaagttctctcgatcagggcatacatctttcttcgacaaggggcagt
aaatggctttgacccagctcctcctcctcctggcctgggctcctcgcgcccgttgtcag
cacccggtatgctgccgctcagtgtgtgagtgcccggcctccggggctcctgccctcct
ccaacaacccaggacacccacgcctcacccctcggtgcctgggcccagccctgtgcccc
tccatctgcctccccacacggctggcagagggcaggctgcatgcagtggcggtgcttcg
gccctgcccagccccaggactctgcgcgatatcaatactggctatttctcttctcgcc
gtagtgccgttggtttcacatgattgcacttttgtgggtcacgaggtgatacatacttg
tattacttggtcactggatgcagaagtacccatttgtcatccctgcctcatagcccccg
ccctgctgtactgataggatttagttgtgtttaggacattgcgaatcttctacaagttc
tcccccaatcaggttgacacataccctcctcctgagcccccgagcccccctgggcgccc
tcagtgctcacgatcatgtgtttcccggccctacccccagtctgggcccgttactgcca
ggagtcaggaaggtcgctgagttagggaatattgtctgtactctcgttttacgtagcag
ttccatccatagactgcctccagagcagtgaaacgccatgctgagcccccctggcaggag
cctcgtgcctgggcacgcacgggctgagcctcaggccatctcctcctccatgtgcctca
gactcgggggaggggtgacggcgtccgtgccagtgtcctgtgcatcctttgattactct
catgctgcatttactgtttacatttgttttattgtacataggtttgtaaacattattgc
ctaagatatttgtatataacttgggctttgtagcttttatttattcagaactcatatgg
catgttaatgactcccgatggtgtcctactctgggcagctgtataggatcatcatgtgg
ttaaaaaaccagttccctcaaaaaaaatcttttaatgtggaaacaataaatttcac)
 and AAB99905 
MDGEDIPDFSSLKEETAYWKELSLKYKQTFQEARDELVEFQEGSRELEAELEAQLVQAE
QRNRDLQADNQRLKYEVEALKEKLEHQYAQSYKQVSVLEDDLSQTRAIKEQLHKYVREL
EQANDDLERAKRATIVSLEDFEQRLNQAIERNAFLESELDEKESLLVSVQRLKDEARDL
RQELAVRERQQEVTRKSAPSSPTLDCEKMDSAVQASLSLPATPVGKGTENSFPSPKAIP
NGFGTSPLTPSARISALNIVGDLLRKVGALESKLAACRNFAKDQASRKSYISGNVNCGV
MNSNGTKFSRSGHTSFFDKGAVNGFDPAPPPPGLGSSRPLSAPGMLPLSV),
 for the cDNA and the corresponding protein, respectively.
   This approximately 2.2 kb long cDNA was completely sequenced, and the sequence was deposited at GenBank under the Acc. No. AF217798 
(agaatggcctcggacacccaggcagtccctgacgtgtcggggaggagccgggcgcgga
ggtacgctgagtggagctcggggctgcgtaggggagctgagccgagcggctgggcgggc
ctggccgggccagcggaggggagacgtcggttgagcggcggcgaacatgcgcttttgac
acattggaggctttcttgatcatggatggtgaagatataccagatttttcaagtttaaa
ggaggaaactgcttattggaaggaactttccttgaagtataagcaaagcttccaggaag
ctcgggatgagctagttgaattccaggaaggaagcagagaattagaagcagagttggag
gcacaattagtacaggctgaacaaagaaatagagacttgcaggctgataaccaaagact
gaaatatgaagtggaggcattaaaggagaagctagagcatcaatatgcacagagctata
agcaggtctcagtgttagaagatgatttaagtcagactcgggccattaaggagcagttg
cataagtatgtgagagagctggagcaggccaacgacgacctggagcgagccaaaagggc
aacaatagtttcactggaagactttgaacaaaggctaaaccaggccattgaacgaaatg
catttttagaaagtgaacttgatgaaaaggaatctttgttggtctctgtacagaggtta
aaggatgaagcaagagatttaaggcaagaactagcagttcgggaaagacaacaggaagt
aactagaaagtcggctcctagctctccaactctagactgtgaaaagatggactccgccg
tacaagcatcactttctttgccagctacccctgttggcaaaggaacggagaacactttt
ccttcaccgaaagctataccaaatggttttggtaccagtccactaactccctctgctag
gatatcagcactaaacatcgtgggggatctcttacggaaagtaggggctttagaatcca
aattagcagcttgcaggaattttgcaaaggaccaagcatcacgaaaatcctatatttca
gggaatgttaactgtggggtgctgaatggcaatggcacaaagttctctcgatcagggca
tacatctttcttcgacaaaggggcagtaaacggctttgaccccgctcctcctcctcctg
gtctgggctcctcgcgtccatcgtcagcgccgggtatgctgcctctcagtgtgtgagtg
cctagcctccaggtgggggctcctgccctcctccaacaacccaggacacccacgcctca
cccctcggtgcctgggcccagccccgtgcccctccgtctgcctccgcacggctggcaga
gggcaggctgcatgcagtggcggctactgggccctgcccagccccggaactctgcgcga
tatcaatactggctatttctcttctcgccgtagtgccgttggtttcacatgattgcac
ttttgtgggtcgcaaggtgatacatacgtgtattacttggtcactggatgcagaagtac
ccattcatcacacctgccccatagcccccactctgctgtactgataggatttagttgtg
ttttaggacattgcaaatcttctagaagttctcccccaaatcaggtcaatgtgtgccct
cctgagctcccacccaggcatctccagtgctcatgatcatgtgtcccccaactccaccc
ctcacagtttgggcctgtttctggcaaagagtcaggaaggttactgaattagggaacat
tttctgcaccttctgattttacttaagcagctaccattccatggacttgcctcccagag
cagcacaatgcccgtctgagccccacgtggcaggagcctctgggacggggcacacacag
gcccagcctctgtgctgtctcctcctctgtgcgcctcagactcggggtgagggaggcgg
gcagcctctcgccagccttcccgtccttcagttcaacgacatctttggagtgtttttgt
tttctcttccaagggccgtcccgttgtgttaggaagggtgagtggctggttccagggtg
 
ggccggtgccagctccggggtggactgaacagcggcggctgtccctgtgcatcctttga
ttactctcatgctgcatttactgtttacatttgttttattgtacataggtttgtaaaca
ttattgcctgagatatttgtatataacttgggctttgtagcttttatttattcagaacg
catacggcatgttaatgactctgatggtgtcctcctctgggcagctgtataggatcatc
atgtggttacaaaaatacttccctcaaaaaattcttttaatgtggaaacaataaatt
tcacagaaaaaaaaaaaaaaaaaaaaaaaaaa)          and          AAF24516
(MDGEDIPDFSSLKEETAYWKELSLKYKQSFQEARDELVEFQEGSRELEAELEAQLVQA
EQRNRDLQADNQRLKYEVEALKEKLEHQYAQSYKQVSVLEDDLSQTRAIKEQLHKYVRE
LEQANDDLERAKRATIVSLEDFEQRLNQAIERNAFLESELDEKESLLVSVQRLKDEARD
LRQELAVRERQQEVTRKSAPSSPTLDCEKMDSAVQASLSLPATPVGKGTENTFPSPKAI
PNGFGTSPLTPSARISALNIVGDLLRKVGALESKLAACRNFAKDQASRKSYISGNVNCG
VLNGNGTKFSRSGHTSFFDKGAVNGFDPAPPPPGLGSSRPSSAPGMLPLSV),
 for the cDNA and the protein sequences, respectively.
b) messenger RNA was purified from total RNA using pre-packed oligo-dT cellulose columns or the resin in suspension;
c)the quality of the resulting messenger RNA was analyzed by electrophoresis in denaturing agarose gel (containing 1-2.5% of formaldehyde), followed by staining with ethidium bromide or other nucleic acid dyes like "Syber Green" (Molecular Dynamics), followed by photodocumentation and by analysis after hybridizations with specific probes (Northern Blot analysis);
d)messenger RNA was reverse-transcribed generating the corresponding double-strand cDNA that was cloned into a plasmid vector, cosmid or phage vector, in order to allow its "in vitro" amplification;
e) the cDNA insert coding for the EOPA was identified and selected by hybridization, immune-selection using a specific anti-EOPA antibody, or alternatively by detection of EOPA specific activity;
f)the cDNA insert was then amplified and isolated by PCR ("polymerase chain reaction"), or alternatively by amplifying the vector containing this cDNA in bacteria, followed by digestion with restriction enzymes which allow the release of the cDNA from the vector;
g) alternatively, the amplification of the cDNA of choice was performed by direct PCR amplification, using messenger or total RNA and specific oligonucleotides (RT-PCR - reverse transcription polymerase chain reaction);
h) the cDNA was then completely sequenced, allowing the determination of the respective deduced amino acid sequence, which primary and secondary structure was analyzed, including homology search analysis using specific softwares and databanks.

### Expression of the recombinant EOPA

The recombinant EOPA can be expressed using heterologous systems such as, for example, bacteria, yeast, baculovirus, eukaryotic cells in culture, and others. The production of the recombinant EOPA in bacteria was performed after subcloning the cDNA coding for the EOPA in frame into an expression vector (e.g., pProEx-HTc), such that the transcription initiation signal (including the initial methionine) of the vector was used. The *Escherichia coli* bacteria were transfected or transformed with this construction by electroporation or "heat-shock" treatment, and the expression of the respective recombinant protein was induced by the addition of IPTG (isopropyl-thiogalactosidase). The expressed protein was produced either as a recombinant or as a fusion protein, with an anchor protein or a poly-histidine tag sequence, which help in the identification and purification processes of the target protein.

The recombinant protein obtained as described above presented biochemical and immunological characteristics identical to those previously observed for the natural protein present in the brain (Hayashi et al., 2000).

The production of the recombinant EOPA, specifically in bacteria, comprised the following steps:
a) the isolated cDNA, coding for the EOPA, was cloned in frame into a plasmidial expression vector, such that the production of EOPA was achieved as a recombinant molecule or as a fusion protein with anchor proteins or a oligopeptide tag sequence;
b) the process for the production of the recombinant or fusion protein requires the transformation of the host bacteria (*Escherichia coli* strains DH5α, BL21(DE3) or similar) with the plasmidial vector construct, as described above, containing the cDNA insert coding for the EOPA cloned in frame in the expression vector; the transformation can be performed by heat-shock or electroporation of the bacteria, previously prepared using the appropriate protocol for each of these methods;
c) the transformed bacteria, obtained as described above, were amplified by growth in appropriate culture medium (usually LB medium containing appropriate antibiotics for selection), until obtaining the optic density lectures of about 0.6 at wavelengths between 560 and 600 nm; at this point, the production of the target protein was induced by addition of expression induction compounds, e.g. IPTG (isopropyl-thiogalactosidase), to a final concentration of about 0.5 to 1 mM, followed by incubation at 30°C or 37°C in a shaker;
e) when the protein was obtained as a fusion protein, it was separated from other soluble bacterial proteins (contaminants) using affinity columns, containing immobilized nickel for poly-histidine fusions or glutathione-sepharose resin for fusions with GST (glutathione-S-transferase); subsequently, the recombinant protein was obtained by cleaving off the anchor protein or polypeptide through digestion with specific proteases (like thrombin, factor X, enterokinase, etc), whose cleavage sites had been introduced between the anchor protein and the recombinant protein of choice;
f) the purity and the quality of the recombinant protein was evaluated by electrophoresis in polyacrylamid gel (SDS/PAGE), Western Blot, mass spectrometry, and determination of the specific activity by enzymatic kinetics, using natural and synthetic peptide substrates.

### Determination of the gene sequence of the human EOPA

The complete sequence of the human gene coding for EOPA was determined by the sequence analysis of the human genome entirely accessible in public data bases. The *EOPA* gene comprises 9 exons and 10 introns, and is approximately 40 kb long. This gene is located at chromosome 17p12.9, relatively close to the locus of the p53 gene (separated by approximately 0.7 cM), and on the same arm of chromosome 17 on which the gene *LIS1* is located (17p13.3). In the upstream region of the EOPA gene, including the promoter region, some transcription regulatory factors binding sites were found, such as for: AP1, cMyb, SP1, nMyc, cMyc, among others.

### Analysis of the promoter region of the gene encoding for EOPA

Analysis of the promoter region of the gene coding for the rabbit EOPA identified some of the main transcriptional factors responsible for the regulation of the expression of this enzyme. Based on the luciferase activity measured for the obtained constructions, the region between the positions -725 and -648 has at least a site recognized by a transcription factor responsible for a strong repression of the expression of EOPA in gliomas (lineage C6), probably, recognized by the product of the protooncogene *c-myb* (Thiele et al., 1988). Moreover, this study indicated that the human EOPA gene shows in its upstream region, between the position -980 and -310, recognition sites that regulate positively this gene. Besides the fact that the present work was performed only using the C6 lineage, the obtained results allowed to observe that even showing low similarity for the upstream regions, both human and rabbit EOPA gene seem to be negatively regulate in gliomas, where low EOPA-like peptidase activity have been observed (enzymatic catalytic activity measurements). On the other hand, the segment of the promoter region of the EOPA gene analyzed seem to contain a recognition site for a transcription factor that acts as an activator of the expression of this gene in the studied cell lineage, e.g., C6 cells.

The family of the *myb* genes has been described as a class of oncogenes, which regulate the transcription of gene involved in the cellular proliferation and differentiation (Ness, 1999). This family is composed by three distinct genes: A*-myb,* B*-myb,* and *c-myb* (Nomura et al., 1988; Oh et al., 1999). Between the well-known properties of *c-myb* that is noteworthy to mention that it is a survive factor that inhibits the differentiation of the myeloid and erythroid cells, allowing the blocked immature cells to continue dividing (Weston, 1998). Some groups have suggested a relationship between the *c-myb* gene and the normal function of neuronal cells (Welter et al., 1990), and also of some tumor cells lines (Gozes et al., 1987). However, the physiological function of *c-myb* gene was not determined yet.

### Generation of the anti-EOPA antibodies showing anti-catalytic activity and ability to discriminate functional similar proteins

Polyclonal antibodies were generated in rats, mice and rabbits, using the purified natural EOPA or the active recombinant EOPA, expressed in bacteria, specifically in *Escherichia coli,* for the immunization. Male Balb-C mice, 7 to 8 weeks old, and weighing between 18 and 22 g were immunized with purified natural human EOPA, or catalytically active recombinant enzyme. For each of the four immunizations, 2 µg of the purified protein or 3 µg of the recombinant protein, absorbed on aluminum hydroxide [Al(OH)₃] were injected intradermically, in weekly intervals. Blood samples were collected one week after the last immunization, and the serum was stored at -20°C. The antiserum titer was evaluated by ELISA, using the appropriate antigen. The anti-catalytic activity of the obtained antiserum was evaluated by HPLC or fluorimetrically, using the quenched fluorescent peptides substrates. Before use, the antiserum was pre-heated at 55°C, for 5 minutes, in order to eliminate any contaminating peptidase activity. Tests to standardize the use of the antibody showed that the incubation of the anti-EOPA antibody for 30 minutes, at 37°C, with the recombinant EOPA reached the maximum inhibitory efficiency, and that this inhibition was specific, since the incubation with other tissue oligopeptidases, such as the thimet oligopeptidase (TOP) and neurolysin (NL), did not show any inhibitory activity. Using this antibody, it was possible to discriminate the activity of the EOPA from functionally similar, but not homologous enzymes, such as the TOP and the NL, which are also present in animal tissues and cells lineages.

### Proteolytic properties and biochemical characteristics of the EOPA

Kinetic assays using natural peptide substrates like bradykinin, neurotensin and opioid peptides, composed by 9 to 13 amino acid residues, were essential for the discovery and characterization of the EOPA in our laboratory (see the summary of this literature in Camargo et al., 1994). Since 1990, we have developed peptide substrates derived from natural substrates with quenched fluorescence, standardizing the characterization of this enzyme, simplifying and reducing the costs, and making the characterization of the kinetic properties of the EOPA more reliable (Juliano et al., 1990). The peptide substrates with quenched fluorescence (qf) used have the radical Abz (*ortho*-aminobenzoic acid) at the amino terminus of the peptide, and the EDDnp [N-(2,4-dinitrophenyl) ethylenediamine] at the carboxyl terminus of the peptide. The amino acid residues of these peptides are abbreviated by the one letter code, as follow:

| | |
|---|---|
| G-glicina | N-asparagina |
| A-alanina | Q-glutamina |
| P-prolina | D-ácido aspártico |
| V-valina | E-ácido glutâmico |
| I-isoleucina | K-lisina |
| L-leucina | R-arginina |
| S-serina | F-fenilalanina |
| T-treonina | H-histidina |
| W-triptofano | Y-tirosina |

Currently, the peptide of choice used for the determination of the EOPA activity is Abz-GFAPFRQ-EDDnp (SEQ ID NO: 4). This method of characterization of the natural EOPA was applied to the recombinant enzyme, completely reproducing the results obtained with the natural EOPA (Hayashi *et al.,* 2000). This method for enzymatic assay was largely employed to test several peptide substrates with quenched fluorescence, which was used to characterize several tissue oligopeptidases such as the EOPA, TOP and NL (Oliveira et al., 2001).

The natural protein, as well as the recombinant form, hydrolyzes peptides of 7 to 13 amino acid residues selectively, and their isoelectric point is between 5.22 and 5.50 (reviewed by Medeiros, 1992). This enzyme specifically hydrolyzes the Phe⁵-Ser⁶ bond of bradykinin (Camargo et al., 1973), and the Arg⁸-Arg⁹ bond of neurotensin (Camargo et al, 1983), and also releases [Met⁵]- or [Leu⁵] -enkephalin from several opioid peptides, which contain enkephalin in their sequences (Camargo et al., 1985; 1987), suggesting its possible involvement in the biotransformation of bioactive peptides.

The EOPA is a thiol-activated endopeptidase, insensible to the EDTA, presenting a molecular mass of approximately 40 kDa, and can be found associated to other cytosolic proteins, generating complexes with higher molecular masses. This last aspect was verified by gel filtration of the cytosol of animal tissues, specifically the cytosol of rat brain, performed using a Superose 12 column (Amersham Bioscience) calibrated with known molecular weight standards. The proteins present in the efflux were monitored by measuring the enzymatic activity, Western blot analysis and ELISA, using the anti-EOPA antibodies.

### Inhibitors of the EOPA

The state of the art has shown by kinetic assays that EOPA is a thiol-activated enzyme, since it is activated by the reducing agent dithiothreitol (DTT), and also inhibited by thiol agents like the p-chloromercuribenzoate (PCMB) and the 5'-dithio-bis-(2-nitrobenzoic acid) (DTNB), classic inhibitors of the cysteinyl proteases (Oliveira et al., 1976; Camargo et al., 1983; Hayashi et al., 1996 and 2000). Later, using mimetic compounds of the enzyme substrates (derivatives of the dynorphin), which were labeled with a thiol-reactive group Npys (S-(3-nitro-2-pyridine sulfenyl), the existence of a critical cysteine residue close to the catalytic site was demonstrated (Gomes et al., 1993; Hayashi et al., 1996). This critical cysteine residue of EOPA reacted irreversibly with the site-directed inhibitor, thereby corroborating with the hypothesis that this enzyme is a cysteine protease.

Therefore, EOPA is a thiol-dependent endooligopeptidase, insensible to the EDTA. However, it can be inhibited by compounds specially designed to inhibit metalloproteases, like the JA2 (Shrimpton et al., 2000), but not by the phosphinic peptide inhibitor synthesized by Jiracek and collaborators (1995). The JA2 inhibitor was obtained by modifications in the structure of the cFP, a specific inhibitor for TOP, but soon after it was verified by the inventors that both compounds inhibited the EOPA activity with a similar Kᵢ of approximately 17 nM.

**Inhibition of the endooligopeptidases by JA2 and the anti-EOPA antibody**

| **Inhibitor / Enzyme** | **rEOPA** | **Thimet oligopeptidase** | **neurolysin** |
|---|---|---|---|
| JA2 Anti- EOPA | Kᵢ = 17 nM 100% of inhibition | *Kᵢ = 23 nM n.i. | Kᵢ > 200 nM n.i. |

| | | | |
|---|---|---|---|
| * Shrimpton et al., 2000 | | | |

### Distribution of EOPA in rat tissues

The expression of the EOPA in distinct rat tissues and cells was analyzed by biochemical and immunological methods, and also by employing molecular biology techniques, which means by evaluating/quantifying the specific catalytic activity, immunohistochemistry, hybridization *"in situ"* and Northern blot analysis. Using these assays, it was possible to verify a higher expression of EOPA in the brain, and show strong hybridization signals mainly in some layers of the cortex, hippocampus, cerebellum, and the basal nucleus of Meynert, indicating that the transcription level of the specific messenger RNA is in these regions was higher.

Furthermore, tissular and cellular distribution studies by immunohistochemistry has demonstrated the co-localization of EOPA with opioid peptides and their precursors in the central nervous system, and in the cellular body and neuronal axons of the retina of vertebrates, which are rich in [Leu⁵] -enkephalin, as previously shown (Oliveira et al., 1990; Paik et al., 1992; Ferro et al., 1991; Hayashi et al., 2000). There are also evidences that the EOPA is secreted into the extracellular space, like other peptide messenger metabolizing enzymes.

Among the tissues (brain, testes, heart, spleen, liver, lung, skeletal muscle, and kidney) analyzed for the proteolytic activity, we observed that the cytosol of kidney showed the lowest EOPA and neurolysin activity levels. On the other hand, the thimet oligopeptidase (TOP) participates with 32 % of the total activity in this tissue, when the substrate Abz-GFAPFRQ-EDDnp (SEQ ID NO: 4) was used, showing an ubiquitous distribution of this enzyme. In general manner, the enzymatic activity of EOPA and thimet oligopeptidase is homogenously distributed in the cytosol of the most of tissues, with exception for the cytosol of the brain and kidney

EOPA is preferentially found in the brain cytosol, accounting for 60% of the total oligopeptidase activity observed in this tissue. The activity of the thimet oligopeptidase was not detected in this cytosol, while 20% of the activity observed in this cytosol is due to neurolysin, and 20% due to the activity of other cytosolic enzymes, which hydrolyze this substrate.

In order to better demonstrate the characteristics identified above, which are objects of the present invention, the table above shows the distribution of EOPA, determined by the specific activity of the anti-EOPA antiserum, on the percentage of hydrolysis of the fluorescent substrate Abz-GFAPFRQ-EDDnp (SEQ ID NO: 4) by the cytosolic endooligopeptidases.

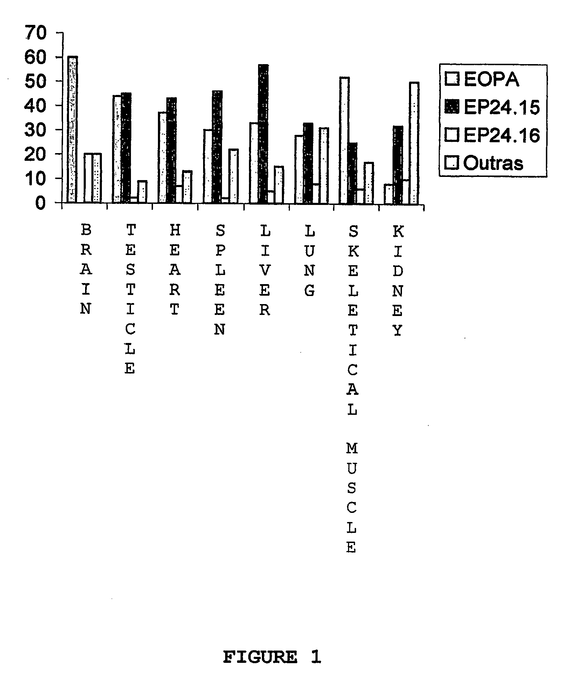

Additionally it can also be seen in figure 1, that refers to the percentage of endoologopeptidase activities with the substrate Abz-GFAPFRQ-EDDnp (SEQ ID NO: 4).

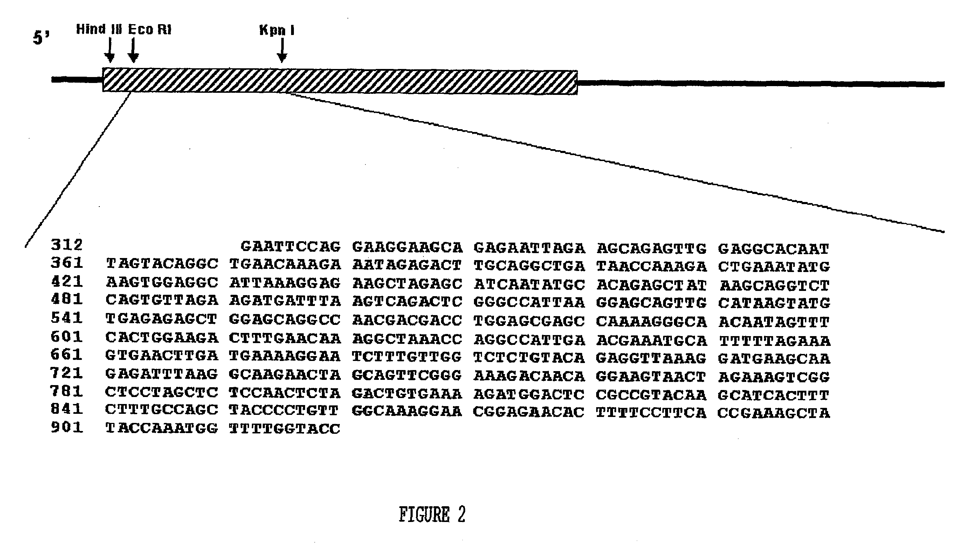

Figure 2 is a schematic representation of the complete sequence of cDNA coding for human EOPA where is detached the segment used to the generation of the employed probes in the EOPA distribution study for hybridization "in situ".

**Percentage of hydrolysis of the fluorescent substrate Abz-GFAPFRQ-EDDnp by the cytosolic endooligopeptidases.**

| **Cytosol** | **EOPA** | **Metalooligopeptidases** **TOP e NL*** | **Others** |
|---|---|---|---|
| Brain | 60% | 20% | 20% |
| Testis | 44% | 47% | 9% |
| Heart | 37% | 50% | 13% |
| Spleen | 30% | 48% | 22% |
| Liver | 33% | 62% | 15% |
| Lung | 28% | 41% | 31% |
| Skeletal muscle | 52% | 31% | 17% |
| Kidney | 8% | 42% | 50% |

| | | | |
|---|---|---|---|
| * The enzymatic activity of TOP and NL was deduced from the percentage of inhibition of the cytosolic peptidases by the phosphinic inibidors, Z-(L, D)-F-ψ-(PO₂CH₂)- (L, D) -A-R-M e P-F-ψ- (PO₂CH₂) -L-P-NH₂, which are specific for the TOP and NL, respectively (Jiracek et al., 1995). | | | |

### Expression of EOPA in neuronal cells and melanomas

"*In vitro"* neuronal differentiation from pluripotent stem cells has been widely accepted as a model for basic studies of the molecular mechanisms involved in the initial steps of embryogenesis. Studies on the expression and function of EOPA have been performed with the P19 cells lineage, derived from murine embryonal carcinoma, as a model for "*in vitro"* neuronal differentiation process. Embryonic stem cells derived from the central layer of the blastocysts have been used to studying the control of the gene expression. They are also potential candidates as therapeutic agents to the treatment of neuronal degenerations and for dopaminergic neuron transplants employed to the treatment of Parkinson's disease. Embryonic carcinoma cells are easily manipulated in culture, normally obtained from neoplasias of germinal tissues. They behave like stem cells, and present a gene expression pattern similar to that of the cells found in the inner layer of the epiblasts, and in culture, can originate typical endodermic, mesodermic or even ectodermic cells (Martin, 1980). The totipotent P19 cells (McBurney et al., 1982), derived from murine embryonic carcinoma were used as differentiation model, and also in studies which aim is the identification of the necessary factors for their irreversible commitment to the distinct differentiation pathways. Undifferentiated P19 cells are characterized by a high proliferation rate, and both cultures with high densities of adhered or in suspension cells induce the differentiation process. This process can also be triggered by chemical factors, such as the retinoic acid or DMSO. For instance, it is known that, in the presence of 10⁻⁷ M retinoic acid, the P19 cells differentiate into neuroectodermic cells (Jones-Villeneuve et al., 1982).

In protocols based on preventing adhesion, cellular aggregates are maintained in suspension for five days, originating the following cellular types: on the seventh day neurons appear, and on the tenth day astrocytes and glial cells can be observed. Accordingly, variation in the nature and concentration of the chemical inducer may induce the P19 cells into multiple differentiation pathways leading to the formation of smooth or skeletal muscle cells, neurons or astrocytes (Edward and McBurney, 1983). Studies performed by the inventors, demonstrated an increased expression of the EOPA messenger RNA in the embryonic corpuscles and in the neuronal differentiating cells, approximately on the eighth day after the treatment with retinoic acid, when the formation of neurites is observed. The aim of this work is to study the physiological role of EOPA and to determine the mechanisms of its genetic and protein modulation during the process of neuronal differentiation.

The PC12 lineage cells are derived from a rat tumor (transplantable rat pheochromocytoma) that reversibly responds to nerve growth factor (NGF), which induces to a neuronal phenotype, and poorly adheres to plastic and tend to grow forming small clusters (Greene et al., 1976). When maintened in culture under normal conditions, these cells are similar, in both biochemical and morphological aspects, to chromaphinic cells of fetal adrenal glands. They can differentiate in two ways: 1) into chromaphinic-like cells in response to glucocoticoids, and 2) into sympathic neuronal cells in response to neurotrophic factors like NGF or fibroblast growth factors (FGF). Furthermore, these cells express several bioactive peptides like the enkephalins, dynorphins and neurotensin.

These properties turn this lineage into an experimental model for the study of enzymes involved in the biotransformation of neuropeptides. We have previously demonstrated that the proteolytic activity of the EOPA was present in the cytosol of these cells, and that this activity was modulated in response to the addition of cAMP, and not in response to the treatment with FGF.

On the other hand, it is known that the treatment of these cells with FGF determines their differentiation into a neuronal phenotype, and axonal extensions can be observed. Then, the EOPA present in these cells migrates to the ends of the neurites, suggesting a possible role of this enzyme in the formation of these cellular extensions, or in the formation of connections with other cells. Efforts have been done in order to clarify the real role of this protein in these cells.

In order to create a new biological model to study the physiological roles of EOPA, the process of differentiation of stem cells into neurons was standardized.

The embryonic stem cells (ES) are undifferentiated cells, derived from the inner cell mass of blastocysts, and are known to be pluripotent (Evans and Kaufman, 1981; Martin, 1981). Pluripotency is the remarkable capacity of ES cells to resume normal development within an organism, being able to populate different tissues including the germ line. Once cultivated under specific conditions, the stem cells can be maintained at the undifferentiated form during several cellular divisions, even after many passages. On the other hand, these cells can be induced to initiate an "in vitro" differentiation program. For instance, when cultivated in suspension, the stem cells spontaneously form cellular aggregates of differentiated cells named embryoid bodies, which are similar to a pre-implantation embryo. The morphological, immunohistochemical and molecular analysis show that several distinct embryonic lineage cells can be found inside an embryoid body, such as hematopoietic, neuronal, endothelial and muscular cells. These stem cells properties were the stimulus for the use of these cells as "in vitro" model for the early embryonic development, and for this reason is a very powerful tool to answer key questions as, for instance: what is the real role of EOPA in the process of nuclear migration in neuronal cells during the embryogenesis and, moreover, what is the importance of the catalytic activity of EOPA in this process, and how is the intracellular distribution of EOPA in the undifferentiated cells, and in the neuronal precursor cells and mature neurons.

The undifferentiated embryonic stem cells (USP-1 lineage) were cultured in medium containing: DMEM, supplemented with 15% of bovine fetal serum, 100 mg/ml of penicillin/streptomycin and 2 mM of L-glutamine, 1% of non-essential amino acids, 10⁻⁴ M of monothioglicerol, and LIF (Leukemia Inhibitory Factor), in a final concentration of 1,000 units/ml. All the undifferentiated stem cells culture was maintained over a primary culture of previously irradiated murine fibroblasts (Neo Mef2). The differentiation was done after the formation of the embryoid bodies, where 750 cells were maintained in 20 µl of the culture medium drops over a Petri dish, during four days at 37°C, under 5% carbonic gas (during this period the cells form the cellular aggregates known as embryoid bodies). After the embryoid bodies formation, the cells were transferred to new dishes containing the culture medium described above, without the LIF and containing 0.1 to 0.5 µM retinoic acid (used as an agent for induction of the neuronal differentiation). The culture was then maintained with the differentiation inductor agent during four days, when the agent was removed and the cell culture was transferred to new dishes pre-treated with 0.22% gelatin and containing glass slides, where the cells were cultured for additional four days under the same conditions described above, but using neurobasal medium (a specific medium for neuronal cells culture, containing a lower percentage of bovine fetal serum). The selection of the neuronal precursor cells and the mature neurons was done by keeping the cells in the neurobasal medium. After completing the differentiation of the stem cells, the differentiated cells were counted and photographed using a confocal microscopy.

A population of cells differentiated into neurons was obtained following the protocol described above, and the influence of the inhibition of the EOPA catalytic activity during the differentiation and maturation process of these neurons was analyzed after the treatment of these cells with the inhibitor JA2. The treatment of the neuronal precursor cells with this inhibitor led to an inhibition of the neurites formation and induced abnormal differentiation of the cells.

### Role of the EOPA in the formation of the central nervous system during embryogenesis

The current view of the non-proteolytic function of EOPA is that it interacts with other cytosolic proteins through its "coiled-coil" structure, for instance, with Lis1 and Disc1. Lis1 is a member of the WD-40 family of proteins, which has this motif widely repeated (Neer et al., 1994), and acts as intracellular protein that maintains the functional machinery together performing several cellular functions (Hatakeyama et al., 1999; Kitagawa et al., 1999; Sidow et al., 1999). The interaction of the EOPA with Lis1 and Disc1 is essential for the formation of the central nervous system during the embryogenesis, and for vital functions such as the intracellular transport, neuritogenesis, nuclear mobility, and plasticity of the nervous tissue. Interaction anomalies cause neurological diseases as the lissencephaly and the Miller-Diecker syndrome (Reiner et al., 1993; Cardoso et al., 2002). Recently, another pathology describing EOPA participation is the schizophrenia. Schizophrenic patients produce a truncated or mutant form of Disc1 that does not bind to EOPA, and this anomaly seems to be linked to the triggering of the disease (Ozeki et al., 2003; Taylor et al., 2003).

*"In situ"* hybridization studies, employing newly born rats or embryos brain slices, indicated an increase of the transcription level of the EOPA messenger RNA, mainly in the cortex of the newly born, 5 to 10 days-old embryos.

Furthermore, the inventors identified and demonstrated a high evolutionary conservation of the "coiled-coil" domain of the EOPA expressed in distinct animals, such as human, rabbit, mice and rat, in which the same protein was also named Nude-L or Nude-Like or Nude2 (Niethammer et al.,2000; Sasaki et al., 2000; Sweeney et al., 2001).

The helical structure and its ability to interact with other proteins seem to be related to the nuclear and neuronal migration, suggesting an important function for this structure in the process of cellular movement, which occurs mainly during embryogenesis.

Our results proved that the proteins homologous to EOPA, isolated from rat (Nude2, GenBank Acc. No. NM_133320) and from mice (Nude-L, GenBank Acc. No. AF323918), also show EOPA-like peptidase activity when peptide substrates previously employed for EOPA were used (Hayashi et al., 2000), besides showing a blockage of their proteolytic activity by EOPA specific inhibitors. These proteins seem to constitute a new protein family, presenting a highly conserved N-terminus domain, characterized by the presence of a "coiled-coil" structure, and a more divergent C-terminal domain, still presenting fragments of conserved sequences, even in organisms so distant in the evolutionary scale as, for instance, the *Aspergillus* [GenBank Acc. No. AF015037] (Sweeney et al., 2001; Kitagawa e cols., 2000).

The tissue-specific expression of EOPA could be observed in frog embryos through assays using the promoter region of the rabbit EOPA gene regulating the expression of a reporter protein, the GFP (Green Fluorescent Protein). First signs of transcription of the reporter gene were observed in embryos at the stage 13 to 15, and could be followed until approximately stage 40, when the embryo could not be maintained any more. In these experiments, the transcription factors, which regulate the expression of the studied gene, interact with specific sequences present in the promoter region triggering the expression of the reporter protein that indicate the tissues and the development stages in which the target protein is mainly being expressed. Therefore, these studies showed that the EOPA was mainly being expressed in the beginning of the neuronal tube formation during the neurulation, and in the neuronal ectoderm and prosencephalus. In later stages, a specific labeling of the skeletal muscles cells, which form the embryos tail, could also be observed.

These results were also confirmed by the whole mount *"in situ"* hybridization assays, performed with albino *Xenopus laevis* embryos, which confirmed the existence of EOPA homologous messenger RNAs along the anterior-posterior axis of the dorsal region of the embryos around the stage 19. Apparently, this staining includes neuronal tissues, more precisely the prosencephalon and rhombencephalon, extending to the spinal cord, and including the neural crest. Moreover, considering that the homozygous deleterious mutations of the EOPA gene are non-viable, assays were performed, in which the EOPA was super-expressed in *X. laevis* embryos, by injecting the specific messenger RNA, transcribed "*in vitro",* demonstrating that the over-expression of EOPA seems to interfere in the normal formation of the nervous system. A smaller and misplaced eye was observed, as well as a serious malformation of the animal's brain, suggesting an abnormal lamination on the injected side.

### Role of the EOPA in the antigen presenting process

It is well known that the opioids peptides are also immunomodulating agents (Blalock, 1989), and considering the presence of EOPA in cells of the immune system, it is possible to suggest a role for this enzyme in the immune system (Paik et al., 1992). These data and the fact that the size of the antigens presented by the MHC class I (Engelhard, 1994) coincides with the size of the peptides required for the binding with these endooligopeptidases (from 7 to 13 amino acid residues), lead the researchers of the present invention to investigate its possible participation in the process of self and non-self selection. The participation of the thimet oligopeptidase (TOP) in the process of MHC class I antigen presentation was also previously investigated by the inventors, since the epitopes presented by the MHC class I are oligopeptides of 7 to 13 amino acid residues, and they are also competitive inhibitors of this enzyme. Similar results have been reproduced "*in vitro*" with the recombinant EOPA, although subtle differences between the specificity of the EOPA and TOP were observed. We observed that the lowering in the lymphoproliferation levels after the addition of oligopeptidase inhibitors in the antigen presenting cells is mainly due to the inhibition of EOPA, suggesting that this enzyme may be involved in the MHC class I antigen presentation system route. Since the expression of the MHC class I in the nervous tissue is related to a infectious and degenerative processes (Piehl and Lidman, 2001), and to the plasticity of the central nervous system (Huh et al., 2000), the control of the peptidase or the chaperone activity of EOPA could explain its involvement in these processes, which evidently would be influenced by specific inhibitors of EOPA.

### References:

Blalock, J.E. (1989) A molecular basis for bi-directional communication between the immune and neuroendocrine systems. Physiol. Rev. 69, 1 - 32.
Camargo, A.C., Caldo, H., and Emson, P.C. (1983) Degradation of neurotensin by rabbit brain endooligopeptidase A and endo-oligopeptidase B (prolineendopeptidase). Biochem. Biophys. Res. Commun. 116, 1151 - 1159.
Camargo, A.C., Caldo, H., and Reis, M.L. (1979) Susceptibility of a peptide derived from bradykinin to hydrolysis by brain endo-oligopeptidases and pancreatic proteinases. J. Biol. Chem. 254, 5304 - 5307.
Camargo, A.C., Gomes, M.D., Reichl, A.P., Ferro, E.S., Jacchieri, S., Hirata, I.Y., and Juliano, L. (1997) Structural features that make oligopeptides susceptible substrates for hydrolysis by recombinant thimet oligopeptidase. Biochem. J. 324, 517 - 522.
Camargo, A.C., Gomes, M.D., Toffoletto, O., Ribeiro, M.J., Ferro, E.S., Fernandes, B.L., Suzuki, K., Sasaki, Y., and Juliano, L. (1994) Structural requirements of bioactive peptides for interaction with endopeptidase 22.19. Neuropeptides 26, 281 - 287.
Camargo, A.C., Oliveira, E.B., Toffoletto, O., Metters, K.M., and Rossier, J. (1987) Brain endo-oligopeptidase A, a putative enkephalin converting enzyme. J. Neurochem. 48, 1258 - 1263.
Camargo, A.C., Ribeiro, M.J., and Schwartz, W.N. (1985) Conversion and inactivation of opioid peptides by rabbit brain endo-oligopeptidase A. Biochem. Biophys. Res. Commun. 130, 932 - 938.
Camargo, A.C., Shapanka, R., and Greene, L.J. (1973) Preparation, assay, and partial characterization of a neutral endopeptidase from rabbit brain. Biochemistry 12, 1838 - 1844.
Cardoso, C., Leventer, R.J., Dowling, J.J., Ward, H.L., Chung, J., Petras, K.S., Roseberry, J.A., Weiss, A. M., Das, S., Martin, C.L., Pilz, D.T., Dobyns, W.B., and Ledbetter, D.H. (2002) Clinical and molecular basis of classical lissencephaly: Mutations in the LIS1 gene (PAFAH1B1). Hum Mutat. 19, 4-15.
Dauch, P., Vincent, J. P., and Checler, F. (1991) Specific inhibition of endopeptidase 24.16 by dipeptides. Eur. J. Biochem. 202, 269 - 276.
Edwards, M.K.S, and McBurney, M.W. (1983) The concentration of retinoic acid determines the differentiated cell types formed by a teratocarcinoma cell line. Dev. Biol. 98, 187 - 191.
Engelhard, V.H. (1994) Structure of peptides associated with MHC class I molecules. Curr. Opin. Immunol. 6, 13 - 23.
Evans, M., and Kaufman, M. (1981) Establishment in culture of pluripotetial cells from mouse embryos. Nature 292, 154 - 156.
Ferro, E.S., Hamassaki, D.E., Camargo, A.C., and Britto, L.R. (1991) Endo-oligopeptidase A, a putative enkephalin-generating enzyme, in the vertebrate retina. J. Neurochem. 57, 1643 - 1649.
Ferro, E.S., Tambourgi, D.V., Gobersztejn, F., Gomes, M.D., Sucupira, M., Armelin, M.C., Kipnis, T.L., and Camargo, A.C. (1993) Secretion of a neuropeptide-metabolizing enzyme similar to endopeptidase 22.19 by glioma C6 cells. Biochem. Biophys. Res. Commun. 191, 275 - 281.
Gomes, M.D., Juliano, L., Ferro, E.S., Matsueda, R., and Camargo, A.C. (1993) Dynorphin-derived peptides reveal the presence of a critical cysteine for the activity of brain endo-oligopeptidase A. Biochem. Biophys. Res. Commun. 197, 501 - 507.
Gozes, I., Nakai, H., Byers,M., Avidor, R., Weinstein, Y., Shani, Y., and Shows, T.B. (1987) Sequential expression in the nervous system of c-myb and VIP genes, located in human chromossomal region 6q24. Somat. Cell Mol. Genet. 13, 305 - 313.
Greene, L.A., and Tischler, A.S. (1976) Establishment of a noradrenergic clonal line of rat adrenal pheochromocytoma cells which respond to nerve growth factor. Proc. Natl. Acad. Sci. USA 73, 2424 - 2428.
Hatakeyama, S., Kitagawa, M., Nakayama, K., Shirane, M., Matsumoto, M., Hattori, Higashi, H., Nakano, H., Okumura, K., Onoe, K., Good, R.A., and Nakayama, K.I. (1999) Ubiquitin-dependent degradation of lκBα is mediated by a ubiquitin ligase Skp1/Cull/F-box protein FWD1. Proc. Natl. Acad. Sci. USA 96, 3859 - 3863.
Hayashi, M.A.F., Portaro, F.C.V., Tambourgi, D.V., Sucupira, M., Yamane, T., Fernandes, B.L., Ferro, E.S., Rebouças, N.A., and Camargo, A.C.M. (2000) Molecular and immunochemical evidences demonstrate that endooligopeptidase A is the predominant cytosolic oligopeptidase of rabbit brain. Biochem. Biophys. Res. Commun. 269, 7-13.
Hayashi, M.A.F., Gomes, M.D., Rebouças, N., Fernandes, B. L., Ferro, E.S., and Camargo, A.C.M. (1996) Species specificity of thimet oligopeptidase (EC 3.4.24.15). Biol. Chem. Hoppe-Seyler 377, 283-291.
Hayashi, M.A., Pires, R.S., Rebouças, N.A., Britto, L.R., and Camargo, A.C. (2001) Expression of endo-oligopeptidase A in the rat central nervous system: a non-radioactive in situ hybridization study. Brain Res. Mol. Brain Res. 89, 86 - 93.
Huh, G.S., Boulanger, L.M., Du, H., Riquelme, P.A., Brotz, T.M., and Shatz, C.J. (2000) Functional requirement for class I MHC in CNS development and plasticity. Science 290, 2155 - 2159.
Jacchieri, S.G., Gomes, M.D., Juliano, L., and Camargo, A.C. (1998) A comparative conformational analysis of thimet oligopeptidase (EC 3.4.24.15) substrates. J. Pept. Res. 51, 452 - 429.
Jiracek, J., Yiotakis, A., Vincent, B., Lecoq, A., Nicolaou, A., Checler, F., and Dive, V. (1995) Development of highly potent and selective phosphinic peptide inhibitors of zinc endopeptidase 24-15 using combinatorial chemistry. J. Biol. Chem. 270, 21701 - 21706.
Jones-Villeneuve, E.M.V., McBurney, M.W., Rogers, K.A., and Kalnins, V.I. (1982) Retinoic acid induces embryonal carcinoma cells to differentiate into neurons and glial cells. J. Cell Biol. 94, 253 - 262.
Juliano, L., Chagas, J.R., Hirata, I.Y., Carmona, E., Sucupira, M., Oliveira, E.S., Oliveira, E.B., and Camargo, A.C.M. (1990) A selective assay for endo-oligopeptidase A based on the cleavage of fluorogenic substrate structurally related to enkephalin. Biochem. Biophys. Res. Commun. 173, 647 - 653.
Kitagawa, M., Hatakeyama, S., Shirane, M., Matsumoto, M., Noriko, I., Hattori, K., Nakmichi, I., Kikuchi, A., Nakayama, K.I., and Nakayama, K. (1999) An F-box protein, FWD1, mediates ubiquitin-dependent proteolysis of β-catenin. EMBO J. 18, 2401 - 2410.
Kitagawa, M., Umezu, M., Aoki, J., Koizumi, H., Arai, H., and Inoue, K. (2000) Direct association of Lis1, the lissencephaly gene product, with a mammalian homologue of a fungal nuclear distribution protein, rNUDE. FEBS Letts. 479, 57 - 62.
Martin, G.R. (1980) Teratocarcinomas and mammalian embryogenesis. Science 209, 768 - 776.
McBurney, M.W., Jones-Villeneuve, E.M.V., Edwards, M.K.S., and Anderson, P.J. (1982) Control of muscle and neuronal differentiation in a cultured embryonal carcinoma cell line. Nature 299, 165 - 167.
Medeiros, M.S., Iazigi, N., Camargo, A.C., and Oliveira, E.B. (1992) Distribution and properties of endo-oligopeptidases A and B in the human neuroendocrine system. J. Endocrinol. 135, 579 - 588.
Neer, E., Schmidt, C., Nambudripad, R., and Smith, T. (1994) The ancient regulatory-protein family of WD-repeat proteins. Nature 371, 297 - 300.
Ness, S.A. (1999) Myb binding proteins: regulators and cohorts in transformation. Oncogene 18, 3039 - 3046.
Niethammer, M., Smith, D.S., Ayala, R., Peng, J., Ko, J., Lee, M.S., Morabito, M., and Tsai, L.H. (2000) NUDEL is a novel Cdk5 substrate that associates with LIS1 and cytoplasmic dynein. Neuron 28, 697 - 711.
Nomura, N., Takahashi, M., Matsui, M., Ishii, S., Date, T., Sasamoto, R., and Ishizaki, R. (1988) Isolation human cDNA clones of myb related genes, A-myb and B-myb. Nucleic Acids Res. 16, 11075 - 11089.
Oh, I.H., and Reddy, E.P. (1999) The myb gene family in cell growth, differentiation and apoptosis. Oncogene 18, 3017 - 3033.
Oliveira, E.B., Martins, A.R., and Camargo, A.C. (1976) Isolation of brain endopeptidases: influence of size and sequence of substrates structurally related to bradykinin. Biochemistry 15, 1967 - 1974.
Oliveira, E.S., Leite, P.E., Spillantini, M.G., Camargo, A.C., and Hunt, S.P. (1990) Localization of endo-oligopeptidase (EC 3.4.22.19) in the rat nervous tissue. J. Neurochem. 55, 1114 - 1121.
Oliveira, V., Campos, M., Melo, R.L., Ferro, E.S., Camargo, A.C., Juliano, M.A., and Juliano, L. (2001) Substrate specificity characterization of recombinant metallo oligo-peptidases thimet oligopeptidase and neurolysin. Biochemistry 40, 4417 - 4425.
Ozeki, Y., Tomoda, T., Kleiderlein, J., Kamiya, A., Bord, L., Fujii, K., Okawa, M., Yamada, N., Hatten, M.E., Snyder, S.H., Ross, C.A., and Sawa, A. (2003) Disrupted-in-Schizophrenia-1 (DISC-1): mutant truncation prevents binding to NudE-like (NUDEL) and inhibits neurite outgrowth. Proc Natl Acad Sci USA. 100, 289 - 294.
Paik, S.H., Betti, F., Camargo, A.C., and Oliveira, E.S. (1992) Presence of endo-oligopeptidase (EC 3.4.22.19), a putative neuropeptide-metabolizing endopeptidase in cells of the immune system. J. Neuroimmunol. 38, 35 - 44.
Piehl, F., and Lidman, O. (2001) Neuroinflammation in the rat--CNS cells and their role in the regulation of immune reactions. Immunol. Rev. 184, 212 - 225.
Portaro, F.C., Gomes, M.D., Cabrera, A., Fernandes, B.L., Silva, C.L., Ferro, E.S., Juliano, L., and Camargo, A.C. (1999) Thimet oligopeptidase and the stability of MHC class I epitopes in macrophage cytosol. Biochem. Biophys. Res. Commun. 255, 596 - 601.
Portaro, F.C., Hayashi, M.A., Silva, C.L., and Camargo, A.C. (2001) Free ATP inhibits thimet oligopeptidase (EC 3.4.24.15) activity, induces autophosphorylation in vitro, and controls oligopeptide degradation in macrophage. Eur. J. Biochem. 268, 887 - 894.
Reiner, O., Carrozzo, R., Shen, Y., Wehnert, M., Faustinella, F., Dobyns, W.B., Caskey, C.T., and Ledbetter, D.H. (1993) Isolation of a Miller-Dieker lissencephaly gene containing G protein β-subunit-like repeats. Nature 364, 717 - 721.
Rogister, B., Ben-Hur, T., and Dubois-Dalcq, M. (1999). From neural stem cells to myelinating oligodendrocytes. Mol. Cell. Neurosci. 14, 287 - 300.
Sasaki, S., Shionoya, A., Ishida, M., Gambello, M. J., Yingling, J, Wynshaw-Boris, A., and Hirotsune, S. (2000) A LIS1/NUDEL/cytoplasmic dynein heavy chain complex in the developing and adult nervous system. Neuron 28, 681-696.
Shrimpton, C.N., Abbenante, G., Lew, R.A., and Smith, I. (2000) Development and characterization of novel potent and stable inhibitors of endopeptidase EC 3.4.24.15. Biochem. J. 345, 351 - 356.
Sidow, A., Bulotsk, M.S., Kerrebrock, A.W., Birren, B., Altshuler, D., Jaenisch, R., Johnson, K.R., and Lander, E.S. (1999) A novel member of the F-bos/WD40 gene family, encoding dactylin, is disrupted in the mouse dactylaplasia mutant. Nature Gen. 23, 104 - 107.
Silva, C.L., Portaro, F.C., Bonato, V.L., Camargo, A.C., and Ferro, E.S. (1999) Thimet oligopeptidase (EC 3.4.24.15), a novel protein on the route of MHC class I antigen presentation. Biochem. Biophys. Res. Commun. 255, 591 - 595.
Sondek, J., Bohm, A., Lambright, D.G., Hamm, H.E., and Sigler, P.B. (1996) Crystal structure of a G-protein beta gamma dimer at 2.1 A resolution. Nature 379, 369 - 374.
Sweeney, K.J., Clark, G.D., Prokscha, A., Dobyns, W.B., and Eichele, G. (2000) Lissencephaly associated mutations suggest a requirement of the PAFAH1b heterotrimeric complex in brain development. Mech. Dev. 92, 263 - 271.
Sweeney, K.J., Prokscha, A., and Eichele, G. (2001) NudE-L, a novel Lis1-interacting protein, belongs to a family of vertabrate coiled-coil proteins. Mech. Dev. 101, 21 - 33.
Taylor, M.S., Devon, R.S., Millar, J.K., and Porteous, D.J. (2003) Evolutionary constraints on the Disrupted in Schizophrenia locus. Genomics 81, 67-77.
Thiele, C.J., Cohen, P.A., and Israel, M.A. (1988) Regulation of c-myb expression neuroblastoma cells during retinoic acid-induced differentiation. Mol. Cell Biol. 8, 1677 - 1683.
Vincent, B., Beaudet, A., Dauch, P., Vincent, J.P., and Checler, F. (1996) Distinct properties of neuronal and astrocytic endopeptidase 3.4.24.16: a study on differentiation, subcellular distribution and secretion processes. J. Neuroscience 15, 5049 - 5059.
Welter, C., Henn, W., Theisinger, B., Fischer, H., Zang, K.D., and Blin, N. (1990) The cellular myb oncogene is amplified, rearranged and activated in human glioblastoma cell lines. Cancer Lett. 52, 57 - 62.
Weston, K. (1998) Myb proteins in life, death and differentiation. Curr. Opin. Genet. Dev. 8, 76 - 81.

## Claims

1. An in vitro process for endooligopeptidase A (EOPA) enzyme activity inhibition **characterized by** a contacting said EOPA enzyme with N-[1-(R,S)-carboxy-3-phenylpropyl]-Ala-a-aminoisobutyric acid (Aib)-Tyr-p-aminobenzoate (JA-2).

2. An in vitro process for inhibiting the formation of neurites **characterized by** treating neuronal precursor cells with the EOPA enzyme inhibitor JA-2.

3. An in vitro process for lowering the levels of lymphoproliferation by treating antigen presenting cells with an anti-EOPA antibody or the EOPA enzyme inhibitor JA-2.

## Patentansprüche

1. . *In vitro* Verfahren zur Aktivitätshemmung des Endooligopeptidase A (EOPA) Enzyms, **gekennzeichnet durch** Kontaktieren des genannten EOPA-Enzyms mit *N*-[1-(*R,S*)-carboxy-3-phenylpropyl]-Ala-a-Aminoisobuttersäure(Aib)-Tyr-p-aminobenzoat (JA-2).

2. . *In vitro* Verfahren zur Hemmung der Entstehung von Nervenentzündungen, **gekennzeichnet durch** Behandlung von neuronalen Vorläuferzellen mit dem EOPA-Enzym-Hemmstoff JA-2.

3. . *In vitro* Verfahren zur Senkung der Lymphoproliferationswerte durch Behandlung von Antigen aufweisenden Zellen mit einem anti-EOPA Antikörper oder dem EOPA-Enzym-Hemmstoff JA-2.

## Revendications

1. . Procédé *in vitro* pour l'inhibition de l'activité de l'enzyme endooligopeptidase A (EOPA), **caractérisé par** la mise en contact dudit enzyme EOPA avec du *N*-[1-(*R,S*)-carboxy-3-phénylpropyle]-Ala-a-acide aminoisobutyrique (Aib)-Tyr-*p*-aminobenzoate (JA-2).

2. . Procédé *in vitro* pour l'inhibition de la formation de névrites, **caractérisé par** le traitement de cellules précurseuses neuronales avec l'inhibiteur JA-2 de l'enzyme EOPA.

3. . Procédé *in vitro* pour abaisser les niveaux de lymphoprolifération par le traitement de cellules présentant des antigènes, avec un anticorps anti-EOPA ou l'inhibiteur JA-2 de l'enzyme EOPA.
